# EUROPÄISCHE PATENTSCHRIFT

(11) **EP 0 792 267 B1**
(45) Veröffentlichungstag und Bekanntmachung des Hinweises auf die Patenterteilung: **14.10.1998**
(21) Anmeldenummer: 95937877.9
(22) Anmeldetag: 07.11.1995
(51) Int. Cl.: C07D 239/52, C07D 239/56, A01N 43/54

(54) **2- (2-ALKOXY-6-TRIFLUORMETHYLPYRIMIDIN-4-YL)-OXYMETHYLEN]-PHENYLESSIGSÄUREDERIVATE, VERFAHREN UND ZWISCHENPRODUKTE ZU IHRER HERSTELLUNG UND IHRE VERWENDUNG**
2- (2-ALKOXY-6-TRIFLUOROMETHYL PYRIMIDINE-4-YL)-OXYMETHYLENE]-PHENYL-ACETIC ACID DERIVATIVES, PROCESSES AND INTERMEDIATE PRODUCTS FOR THEIR PRODUCTION AND THEIR USE
DERIVES DE 2- (2-ALCOXY-6-TRIFLUOROMETHYLPYRIMIDIN-4-YLE)-OXYMETHYLENE]-PHENYLE ACIDE ACETIQUE, PROCEDES ET INTERMEDIAIRES DESTINES A LEUR PRODUCTION ET LEUR UTILISATION

(30) Priorität: 17.11.1994 DE 4440930; 21.07.1995 DE 19526661
(43) Veröffentlichungstag der Anmeldung: 03.09.1997
(73) Patentinhaber: BASF AKTIENGESELLSCHAFT, 67056 Ludwigshafen (DE)
(72) Erfinder: KIRSTGEN, Reinhard, D-67434 Neustadt (DE); OBERDORF, Klaus, D-69117 Heidelberg (DE); SCHÜTZ, Franz, D-67435 Neustadt (DE); THEOBALD, Hans, D-67117 Limburgerhof (DE); HARRIES, Volker, D-67227 Frankenthal (DE)
(86) Internationale Anmeldenummer: EP9504375
(87) Internationale Veröffentlichungsnummer: WO9616047

(56) Entgegenhaltungen:
- EP-A- 0 513 580
- JOURNAL OF HETEROCYCLIC CHEMISTRY, Bd.9, Nr.3, Juni 1972, PROVO US Seiten 513 - 521 ALBERT W. LUTZ ET AL. 'NOVEL 6-TRIFLUOROMETHYL CYTOSINES AND URACILS.'

## Beschreibung

Die vorliegende Erfindung betrifft 2-[(2-Alkoxy-6-trifluormethylpyrimidin-4-yl)-oxymethylen]-phenylessigsäurederivate der Formel I 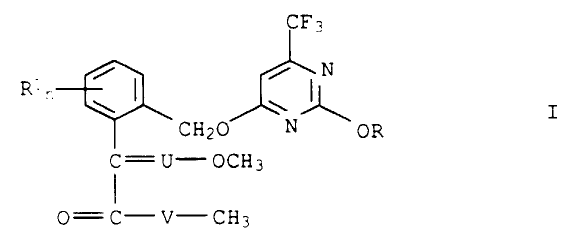 in der der Index und die Substituenten die folgenden Bedeutung haben:
- U: CH oder N;
- V: O oder NH;
- R: C₁-C₆-Alkyl;
- R¹: Cyano, Halogen, C₁-C₄-Alkyl, C₁-C₄-Halogenalkyl, C₁-C₄-Alkoxy, C₁-C₄-Halogenalkoxy und Phenyl;
- n: 0 oder eine ganze Zahl von 1 bis 4, wobei die Reste R¹ verschieden sein können, wenn der Wert von n größer als 1 ist.

Daneben betrifft die Erfindung Verfahren und Zwischenprodukte zur Herstellung dieser Verbindungen und sie enthaltenden Mittel sowie deren Verwendung.

α-Hetaryloxymethylenphenyl-β-methoxy-acrylsäuremethylester werden in der Literatur als Fungizide beschrieben (EP-A 178 826, EP-A 278 595, EP-A 350 691). Außerdem werden in der EP-A 407 873 entsprechende α-[2-(6-Trifluormethylpyrimidin-4-yl)-oxymethylenphenyl]-β-methoxy-acrylsäuremethylester mit acarizider und insektizider Wirkung beschrieben.

Außerdem sind α-[2-(Hetaryloxymethylen)-phenyl]-α-methoxyiminoessigsäuremethylester mit fungizider (EP-A 253 213, EP-A 254 426, EP-A 299 694, EP-A 363 818) und insektizider bzw. acarizider Wirkung (EP-A 407 873) bekannt.

Des weiteren werden in der Literatur α-[2-(Hetaryloxymethylen)-phenyl]-α-methoxyimino-essigsäuremethylamide mit fungizider (EP-A 396 692) und insektizider bzw. acarizider Wirkung (EP-A 477 631) beschrieben.

Die Wirkung der dort beschriebenen Verbindungen gegen tierische Schädlinge ist jedoch in vielen Fällen unbefriedigend.

Der vorliegenden Erfindung lagen daher Verbindungen mit verbesserten Eigenschaften bei der Bekämpfung von Schadpilzen und tierischen Schädlingen, besonders Schadpilze, Insekten, Nematoden und Akaridien, insbesondere Insekten und Akaridien, als Aufgabe zugrunde.

Demgemäß wurden die eingangs definierten Verbindungen I gefunden. Des weiteren wurden Verfahren und Zwischenprodukte zu ihrer Herstellung, sie enthaltende Mittel und Verfahren zu ihrer Verwendung gefunden.

Die Herstellung der Verbindungen I erfolgt in Analogie zu den in der eingangs genannten Literatur beschriebenen Verfahren. Dabei erhält man die Verbindungen I beispielsweise dadurch, daß man ein Pyrimidin-4-ol der Formel II in an sich bekannter Weise in einem inerten organischen Lösungsmittel in Gegenwart einer Base mit einem Benzylderivat der Formel III umsetzt.

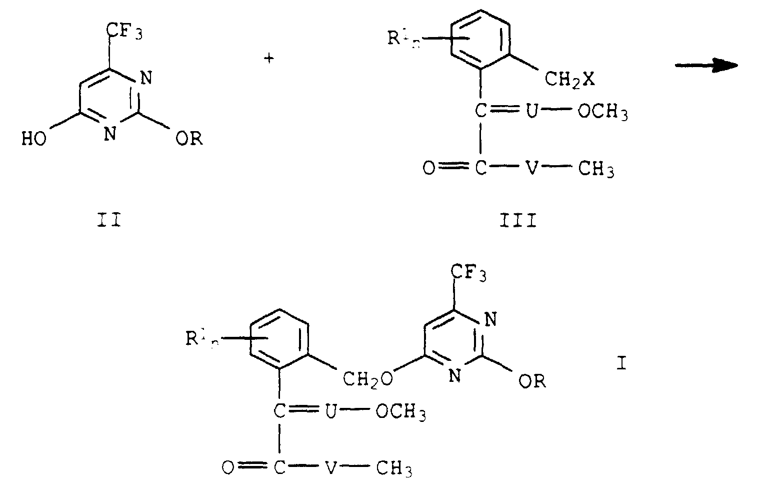

In der Formel III steht X für eine nucleophil austauschbare Abgangsgruppe wie Halogen (z.B. Chlor, Brom und Iod) oder Alkyl- und Arylsulfonyl (z.B. Methylsulfonyl, Trifluormethylsulfonyl, Phenylsulfonyl und 4-Methylphenylsulfonyl).

Diese Umsetzung wird üblicherweise bei Temperaturen von 0°C bis 80°C, vorzugsweise 20°C bis 60°C durchgeführt.

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, Isopropanol, n-Butanol und tert.-Butanol, Ketone wie Aceton und Methylethylketon sowie Dimethylsulfoxid, Dimethylformamid, Dimethylacetatamid, 1,3-Dimethylimidazolidin-2-on und 1,3-Dimethyltetrahydro-2(1H)-pyrimidinon besonders bevorzugt Methylenchlorid, Aceton und Dimethylformamid.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid, Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Kaliumcarbonate und Calziumcarbonat sowie Alkalimetallhydrogencarbonate wie Kaliumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriummethanolat, Kaliumethanolat, Kalium-tert.-butanolat und Dimethoxymagnesium außerdem organische Basen, z.B. tertiäre Amine wie Trimethylamin, Triethylamin, Di-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriumhydroxid, Natriumhydrid, Kaliumcarbonat und Kalium-tert.-butanolat.

Die Basen werden im allgemeinen äquimolar, im Überschuß oder gegebenenfalls als Lösungsmittel verwendet.

Es kann für die Umsetzung vorteilhaft sein, eine katalytische Menge eines Kronenethers wie z.B. 18-Krone-6 oder 15-Krone-5 zuzusetzen.

Die Umsetzung kann auch in Zweiphasensystemen, bestehend aus einer Lösung von Alkali- oder Erdalkalihydroxiden oder Alkali- oder Erdalkalicarbonaten in Wasser und einer organischen Phase wie z.B. halogenierten Kohlenwasserstoffen, durchgeführt werden. Als Phasentransferkatalysatoren können Ammoniumhalogenide und -tetrafluoroborate wie z.B. Benzyltriethylammoniumchlorid, Benzyltributylammoniumbromid, Tetrabutylammoniumchlorid, Hexadecyltrimethylammoniumbromid oder Tetrabutylammoniumtetrafluoroborat sowie Phosphoniumhalogenide wie Tetrabutylphosphoniumchlorid oder Tetraphenylphosphoniumbromid eingesetzt werden.

Es kann für die Umsetzung vorteilhaft sein, zunächst die Verbindungen II mit Base zu behandeln und das resultierende Salz mit den Verbindungen III umzusetzen.

Die Verbindungen II können durch Kondensation von Trifluoracetessigsäureestern VI mit O-Alkylisoharnstoffen VII in Analogie zu bekannten Verfahren [vgl. J. Chem. Soc 1946, 5] erhalten werden.

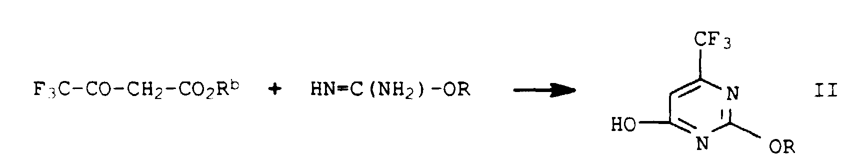

R^{b} in der Formel VI steht für eine C₁-C₄-Alkylgruppe, insbesondere Methyl oder Ethyl.

Die Umsetzung wird üblicherweise bei Temperaturen von 0°C bis 120°C, vorzugsweise 20°C bis 80°C, insbesondere bei der Siedetemperatur des Lösungsmittels. Als Lösungsmittel finden üblicherweise Alkohole, insbesondere Methanol oder Ethanol, Verwendung.

Die O-Alkylisoharnstoffe der Formel VII werden üblicherweise in Form ihrer Salze, insbesondere als Hydrohalogenide (z.B. Hydrochlorid und Hydrobromid) eingesetzt. Bei der Verwendung von Salzen empfiehlt es sich, die Umsetzung in Gegenwart einer Base (z.B. Erdalkalimetall- oder Alkalimetallalkoholate oder -hydroxide wie Natriummethanolat, Natriumethanolat, Kalium-tert.-butylat, Natriumhydroxid, Kaliumhydroxid und Calziumhydroxid) durchzuführen.

Alternativ erhält man die Verbindungen I auch dadurch, daß man ein Sulfonderivat der Formel IV in an sich bekannter Weise [vgl. J. Med. Chem. 27, 1621 (1984; CH-A 649 068] in Gegenwart einer Base mit einem Alkohol der Formel V umsetzt.

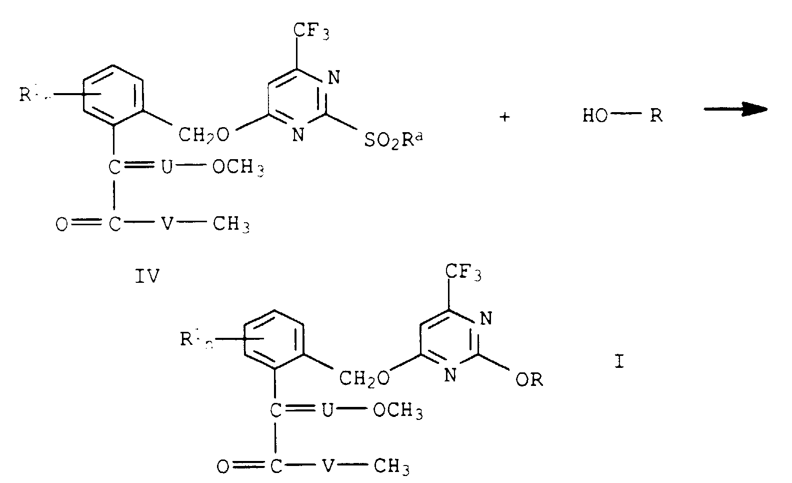

R^{a} in der Formel IV steht für C₁-C₄-Alkyl, insbesondere Methyl.

Als Basen eignen sich insbesondere Natriumhydrid, Kalium-tert.-butylat und Kaliumcarbonat.

Die Umsetzung wird im allgemeinen in einem inerten dipolaren aprotischen Lösungsmittel (insbesondere Dimethylsulfoxyd, Dimethylformamid und 1,3-Dimethyltetrahydro-2(1H)-pyrimidinon) durchgeführt.

Die für die Umsetzung benötigten Sulfonderivate der Formel IV erhält man ausgehend von den entsprechenden Sulfiden VIII durch Oxidation.

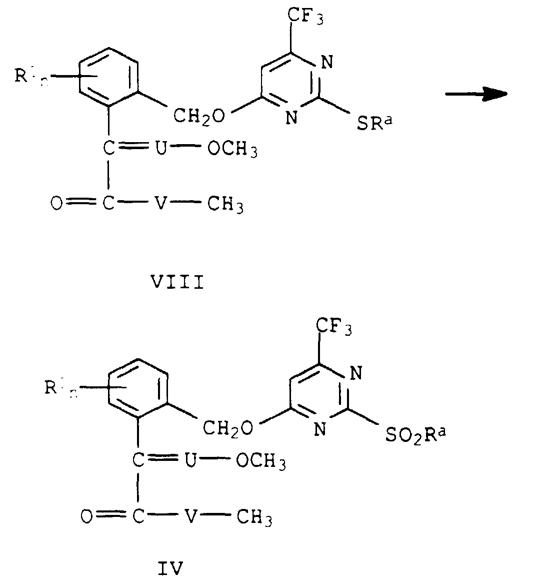

Die Oxidation erfolgt nach aus der Literatur bekannten Verfahren beispielsweise mit Wasserstoffperoxid in konzentrierter Essigsäure [vgl. Chem. Pharm. Bull. 27, 183 (1978)] oder mit Natriumhypochlorid in Wasser [vgl. J. Prakt. Chem. 33, 165 (1966)].

Die Benzylderivate der Formel III, in denen V für Sauerstoff steht, sind aus der eingangs zitierten Literatur bekannt. Benzylderivate II, in denen U für N und V für NH stehen, werden in der DE-A 43 05 502 beschrieben.

Alternativ erhält man die Verbindungen der allgemeinen Formel I, in denen U für N und V für NH steht, durch Aminolyse der entsprechenden Ester (V = O) (vgl. Houben-Weyl Bd. E5, S. 983f.).

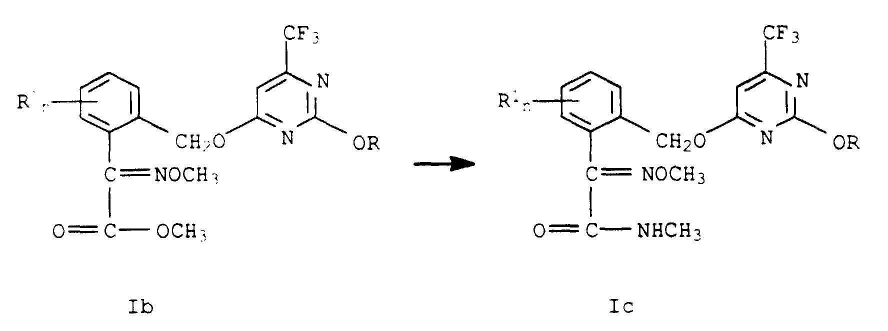

Diese Umsetzung wird üblicherweise bei Temperaturen von 0°C bis 60°C, vorzugsweise 10°C bis 30°C, in einem inerten Lösungsmittel durchgeführt.

Methylamin kann gasförmig eingeleitet oder als wäßrige Lösung zudosiert werden.

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether tert.-Butylmethylether, Dioxan, Tetrahydrofuran und Anisol, Alkohole wie Methanol, Ethanol, n-Propanol, Isopropanol, n-Butanol und tert.-Butanol, besonders bevorzugt Methanol, Toluol und Tetrahydrofuran. Es können auch Gemische der genannten Lösungsmittel verwendet werden.

Bei den in den vorstehenden Formeln angegebenen Definitionen der Symbole wurden Sammelbegriffe verwendet, die allgemein repräsentativ für die folgenden Substituenten stehen:
**Halogen**: Fluor, Chlor, Brom und Iod;
**Alkyl**: gesättigte, geradkettige oder verzweigte Kohlenwasserstoffreste mit 1 bis 6 Kohlenstoffatomen wie Methyl, Ethyl, propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und l-Ethyl-2-methylpropyl;
**Halogenalkyl**: geradkettige oder verzweigte Alkylgruppen mit 1 bis 4 Kohlenstoffatomen (wie vorstehend genannt), wobei diese in Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkoxy**: geradkettige oder verzweigte Alkylgruppen mit 1 bis 4 Kohlenstoffatomen (wie vorstehend genannt), welche über ein Sauerstoffatom (-O-) an das Gerüst gebunden sind;
**Halogenalkoxy**: geradkettige oder verzweigte Halogenalkylgruppen mit 1 bis 4 Kohlenstoffatomen (wie vorstehend genannt), welche über ein Sauerstoffatom (-O-) an das Gerüst gebunden sind; Im Hinblick auf ihre biologische Wirksamkeit sind besondere solche Verbindungen I bevorzugt, in denen R für C₂-C₅-Alkyl, insbesondere C₂-C₄-Alkyl, steht.

Außerdem werden Verbindungen I bevorzugt, in denen n für 0 oder 1, insbesondere 0, seht.

Für den Fall, daß n nicht für 0 steht, werden Verbindungen I bevorzugt, in denen R¹ die folgende Bedeutung hat: Cyano, Fluor, Chlor, Methyl, Trifluormethyl und Methoxy.

Außerdem werden Verbindungen I bevorzugt, in denen ein Rest R¹ in 3-, 4- oder 5-Position des Phenylrings gebunden sind.

Insbesondere sind im Hinblick auf ihre Verwendung die in der folgenden Tabelle zusammengestellten Verbindungen I bevorzugt. Die in der Tabelle für einen Substituenten genannten Gruppen stellen außerdem für sich betrachtet (unabhängig von der Kombination, in der sie genannt sind) eine besonders bevorzugte Ausgestaltung des betreffenden Substituenten dar.

### Tabelle 1

Verbindungen der Formel I, in denen U für CH und V für O steht (≡ Ia) und die Kombination der Substituenten R¹ₙ und R für eine Verbindung einer Zeile der Tabelle A entspricht

### Tabelle 2

Verbindungen der Formel I, in denen U für N und V für O steht (≡ Ib) und die Kombination der Substituenten R¹ₙ und R für eine Verbindung einer Zeile der Tabelle A entspricht

### Tabelle 3

Verbindungen der Formel I, in denen U für N und V für NH steht (≡ Ic) und die Kombination der Substituenten R¹ₙ und R für eine Verbindung einer Zeile der Tabelle A entspricht

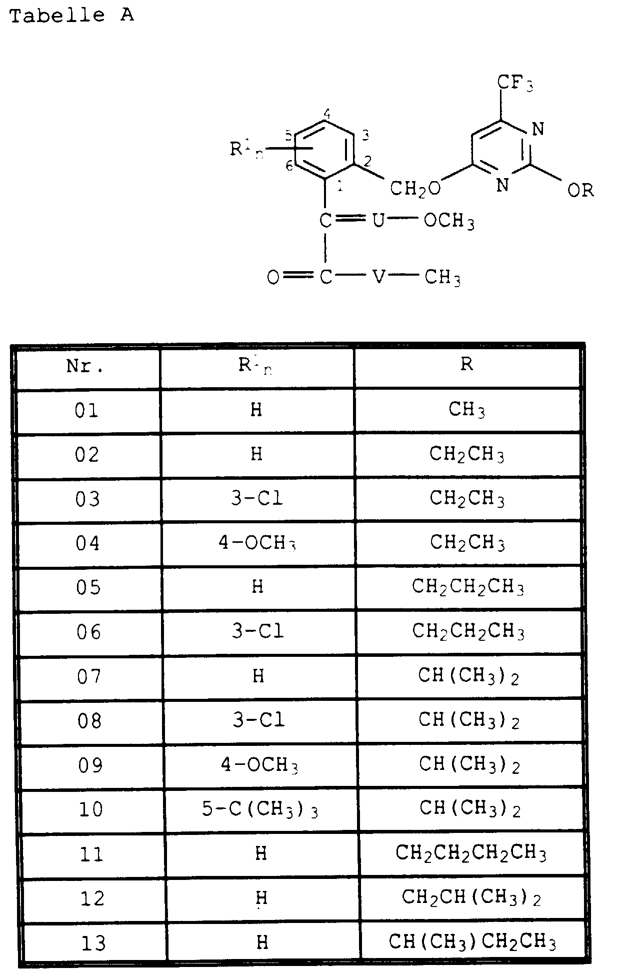

Die erfindungsgemäßen Verbindungen der Formel I eignen sich zur Bekämpfung von tierischen Schädlingen aus der Klasse der Insekten, Akariedien und Nematoden, besonders der Insekten, insbesondere der Akaridien. Sie können im Pflanzenschutz sowie auf dem Hygiene-, Vorratsschutz- und Veterinärsektor als Fungizide und Schädlingsbekämpfungsmittel eingesetzt werden.

Zu den schädlichen Insekten gehören:
aus der Ordnung der Schmetterlinge (Lepidoptera) beispielsweise Adoxophyes orana, Agrotis ypsilon, Agrotis segetum, Alabama argillacea, Anticarsia gemmatalis, Argyresthia conjugella, Autographa gamma, Cacoecia murinana, Capua reticulana, Choristoneura fumiferana, Chilo partellus, Choristoneura occidentalis, Cirphis unipuncta, Cnaphalocrocis medinalis, Crocidolomia binotalis, Cydia pomonella, Dendrolimus pini, Diaphania nitidalis, Diatraea grandiosella, Earias insulana, Elasmopalpus lignosellus, Eupoecilia ambiguella, Feltia subterranea, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera scitella, Lithocolletis blancardella, Lobesia botrana, Loxostege sticticalis, Lymantria dispar, Lymantria monacha, Lyonetia clerkella, Manduca sexta, Malacosoma neustria, Mamestra brassicae, Mocis repanda, Operophthera brumata, Orgyia pseudotsugata, Ostrinia nubilalis, Pandemis heparana, Panolis flammea, Pectinophora gossypiella, Phthorimaea operculella, Phyllocnistis citrella, Pieris brassicae, Plathypena scabra, Platynota stultana, Plutella xylostella, Prays citri, Prays oleae, Prodenia sunia, Prodenia ornithogalli, Pseudoplusia includens, Rhyacionia frustrana, Scrobipalpula absoluta, Sesamia inferens, Sparganothis pilleriana, Spodoptera frugiperda, Spodoptera littoralis, Spodoptera litura, Syllepta derogata, Synanthedon myopaeformis, Thaumatopoea pityocampa, Tortrix viridana, Trichoplusia ni, Tryporyza incertulas, Zeiraphera canadensis, ferner Galleria mellonella und Sitotroga cerealella, Ephestia cautella, Tineola bisselliella;
aus der Ordnung der Käfer (Coleoptera) beispielsweise Agriotes lineatus, Agriotes obscurus, Anthonomus grandis, Anthonomus pomorum, Apion vorax, Atomaria linearis, Blastophagus piniperda, Cassida nebulosa, Cerotoma trifurcata, Ceuthorhynchus assimilis, Ceuthorhynchus napi, Chaetocnema tibialis, Conoderus vespertinus, Crioceris asparagi, Dendroctonus refipennis, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllopertha horticola, Phyllophaga sp., Phyllotreta chrysocephala, Phyllotreta nemorum, Phyllotreta striolata, Popillia japonica, Psylliodes napi, Scolytus intricatus, Sitona lineatus, ferner Bruchus rufimanus, Bruchus pisorum, Bruchus lentis, Sitophilus granaria, Lasioderma serricorne, Oryzaephilus surinamensis, Rhyzopertha dominica, Sitophilus oryzae, Tribolium castaneum, Trogoderma granarium, Zabrotes subfasciatus;
aus der Ordnung der Zweiflügler (Diptera) beispielsweise Anastrepha ludens, Ceratitis capitata, Contarinia sorghicola, Dacus cucurbitae, Dacus oleae, Dasineura brassicae, Delia coarctata, Delia radicum, Hydrellia griseola, Hylemyia platura, Liriomyza sativae, Liriomyza trifolii, Mayetiola destructor, Orseolia oryzae, Oscinella frit, Pegomya hyoscyami, Phorbia antiqua, Phorbia brassicae, Phorbia coarctata, Rhagoletis cerasi, Rhagoletis pomonella, Tipula oleracea, Tipula paludosa, ferner Aedes aegypti, Aedes vexans, Anopheles maculipennis, Chrysomya bezziana, Chrysomya hominivorax, Chrysomya macellaria, Cordylobia anthropophaga, Culex pipiens, Fannia canicularis, Gasterophilus intestinalis, Glossina morsitans, Haematobia irritans, Haplodiplosis equestris, Hypoderma lineata, Lucilia caprina, Lucilia cuprina, Lucilia sericata, Musca domestica, Muscina stabulans, Oestrus ovis, Tabanus bovinus, Simulium damnosum;
aus der Ordnung der Thripse (Thysanoptera) beispielsweise Frankliniella fusca, Frankliniella occidentalis, Frankliniella tritici, Haplothrips tritici, Scirtothrips citri, Thrips oryzae, Thrips palmi, Thrips tabaci;
aus der Ordnung der Hautflügler (Hymenoptera) beispielsweise Athalia rosae, Atta cephalotes, Atta sexdens, Atta texana, Hoplocampa minuta, Hoplocampa testudinea, Iridomyrmes humilis, Iridomyrmex purpureus, Monomorium pharaonis, Solenopsis geminata, Solenopsis invicta, Solenopsis richteri;
aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus hesperus, Lygus lineolaris, Lygus pratensis, Nezara viridula, Piesma quadrata, Solubea insularis, Thyanta perditor;
aus der Ordnung der Pflanzensauger (Homoptera) beispielsweise Acyrthosiphon onobrychis, Acyrthosiphon pisum, Adelges laricis, Aonidiella aurantii, Aphidula nasturtii, Aphis fabae, Aphis gossypii, Aphis pomi, Aulacorthum solani, Bemisia tabaci, Brachycaudus cardui, Brevicoryne brassicae, Dalbulus maidis, Dreyfusia nordmannianae, Dreyfusia piceae, Dysaphis radicola, Empoasca fabae, Eriosoma lanigerum, Laodelphax striatella, Macrosiphum avenae, Macrosiphum euphorbiae, Macrosiphon rosae, Megoura viciae, Metopolophium dirhodum, Myzus persicae, Myzus cerasi, Nephotettix cincticeps, Nilaparvata lugens, Perkinsiella saccharicida, Phorodon humuli, Planococcus citri, Psylla mali, Psylla piri, Psylla pyricol, Quadraspidiotus perniciosus, Rhopalosiphum maidis, Saissetia oleae, Schizaphis graminum, Selenaspidus articulatus, Sitobion avenae, Sogatella furcifera, Toxoptera citricida, Trialeurodes abutilonea, Trialeurodes vaporariorum, Viteus vitifolii;
aus der Ordnung der Termiten (Isoptera) beispielsweise Calotermes flavicollis, Leucotermes flavipes, Macrotermes subhyalinus, Odontotermes formosanus, Reticulitermes lucifugus, Termes natalensis;
aus der Ordnung der Geradflügler (Orthoptera) beispielsweise Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Schistocerca americana, Schistocerca peregrina, Stauronotus maroccanus, Schistocerca gregaria, ferner Acheta domestica, Blatta orientalis, Blattella germanica, Periplaneta americana;
aus der Ordnung der Arachnoidea beispielsweise phytophage Milben wie Aculops lycopersicae, Aculops pelekassi, Aculus schlechtendali, Brevipalpus phoenicis, Bryobia praetiosa, Eotetranychus carpini, Eutetranychus banksii, Eriophyes sheldoni, Oligonychus pratensis, Panonychus ulmi, Panonychus citri, Phyllocoptruta oleivora, Polyphagotarsonemus latus, Tarsonemus pallidus, Tetranychus cinnabarinus, Tetranychus kanzawai, Tetranchus pacificus, Tetranychus urticae, Zecken wie Amblyomma americanum, Amblyomma variegatum, Argas persicus, Boophilus annulatus, Boophilus decoloratus, Boophilus microplus, Dermacentor silvarum, Hyalomma truncatum, Ixodes ricinus, Ixodes rubicundus, Ornithodorus moubata, Otobius megnini, Rhipicephalus appendiculatus und Rhipicephalus evertsi sowie tierparasitische Milben wie Dermanyssus gallinae, Psoroptes ovis und Sarcoptes scabiei;
aus der Klasse der Nematoden beispielsweise Wurzelgallennematoden, z.B. Meloidogyne hapla, Meloidogyne incognita, Meloidogyne javanica, zystenbildende Nematoden, z.B. Globodera pallida, Globodera rostochiensis, Heterodera avenae, Heterodera glycines, Heterodera schachtii, migratorische Endoparasiten und semi-endoparasitische Nematoden, z.B. Heliocotylenchus multicinctus, Hirschmanniella oryzae, Hoplolaimus spp, Pratylenchus brachyurus, Pratylenchus fallax, Pratylenchus penetrans, Pratylenchus vulnus, Radopholus similis, Rotylenchus reniformis, Scutellonema bradys, Tylenchulus semipenetrans, Stock- und Blattnematoden z.B. Anguina tritici, Aphelenchoides besseyi, Ditylenchus angustus, Ditylenchus dipsaci, Virusvektoren, z.B. Longidorus spp, Trichodorus christei, Trichodorus viruliferus, Xiphinema index, Xiphinema mediterraneum.

Die Verbindungen I eignen sich außerdem als Fungizide.

Die Verbindungen I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung des ortho-substituierten Benzylesters einer Cyclopropancarbonsäure gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Sie können in die üblichen Formulierungen übergeführt werden, wie Lösungen, Emulsionen, Suspensionen, Stäube, Pulver, Pasten oder Granulate. Die Anwendungsformen richten sich dabei nach dem jeweiligen Verwendungszweck; sie sollten in jedem Fall möglichst die feinste Verteilung der Wirkstoffe gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können.

Als Hilfsstoffe kommen dafür im wesentlichen in Betracht:
- Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser;
- Trägerstoffe wie natürliche Gesteinsmehle (z.B. Kaoline, Tonerden, Talkum, Kreide) und synthetische Gesteinsmehle (z.B. hochdisperse Kieselsäure, Silikate);
- Emulgiermittel wie nichtionogene und anionische Emulgatoren (z.B. Polyoxyethylen-Fettalkohol-Ether, Alkylsulfonate und Arylsulfonate) und
- Dispergiermittel wie Ligninsulfit-Ablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden.

Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe. Die Wirkstoffkonzentrationen in den anwendungsfertigen Zubereitungen können in größeren Bereichen variiert werden.

Ganz allgemein enthalten die Mittel zwischen 0,0001 und 95 Gew.-% Wirkstoff.

Formulierungen mit mehr als 95 Gew.-% Wirkstoff können mit gutem Erfolg im Ultra-Low-Volume-Verfahren (ULV) ausgebracht werden, wobei sogar der Wirkstoff ohne Zusätze verwendet werden kann.

Für die Anwendung gegen tierische Schädlinge kommen Formulierungen mit 0,0001 bis 10 Gew.%, vorzugsweise 0,01 bis 1 Gew.% Wirkstoff, in Betracht.

Die Wirkstoffe werden normalerweise in einer Reinheit von 90 % bis 100 %, vorzugsweise 95 % bis 100 % (nach NMR-Spektrum) eingesetzt.

Beispiele für solche Zubereitungen sind:
I. eine Lösung aus 90 Gew.-Teilen einer erfindungsgemäßen Verbindung I und 10 Gew.-Teilen N-Methyl-a-pyrrolidon, die zur Anwendung in Form kleinster Tropfen geeignet ist;
II. eine Lösung von 20 Gew.-Teilen einer erfindungsgemäßen Verbindung I in einer Mischung aus 80 Gew.-Teilen alkyliertem Benzol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes von 40 Mol Ethylen oxid an 1 Mol Ricinusöl; durch feines Verteilen der Formulierung in Wasser erhält man eine Dispersion.
III. eine Lösung von 20 Gew.-Teilen einer erfindungsgemäßen Verbindung I in einer Mischung aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl; durch feines Verteilen der Formulierung in Wasser erhält man eine Dispersion.
IV. eine wäßrige Dispersion aus 20 Gew.-Teilen einer erfindungsgemäßen Verbindung I, in einer Mischung aus 25 Gew.-Teilen Cyclohexanon, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl; durch feines Verteilen der Formulierung in Wasser erhält man eine Dispersion.
V. eine in einer Hammermühle vermahlene Mischung aus 20 Gew.- Teilen einer erfindungsgemäßen Verbindung I, 3 Gew.-Teilen des Natriumsalzes der Diisobutylnaphtalin-a-sulfonsäure, 17 Gew.-Teilen des Natriumsalzes einer Ligninsulfonsäure aus einer Sulfitablauge und 60 Gew.-Teilen pulverförmigem Kieselsäuregel; durch feines Verteilen der Mischung in Wasser erhält man eine Spritzbrühe;
VI. eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung I, 92 Gew.-Teilen pulverförmigem Kieselsäuregel und 8 Gew.Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen einer erfindungsgemäßen Verbindung I, 10 Gew.-Teilen des Natriumsalzes eines Phenosulfonsäure-harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkohol-polyglykol-ether, 2 Gew.-Teilen des Natriumsalzes eines Phenolsulfonsäure-harnstoff-formaldehyd-Kondensates und 68 Gew.-Teilen eines paraffinischen Mineralöls;
X. eine in einer Hammermühle vermahlene Mischung aus 10 Gew.-Teilen einer erfindungsgemäßen Verbindung I, 4 Gew.-Teilen des Natriumsalzes der Diisobutylnaphthalin-a-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhält man eine Spritzbrühe, die 0,1 Gew.% des Wirkstoffs enthält.

Die Verbindungen I werden angewendet, indem man die Schädlinge oder Schadpilze oder die vor Schädlingen oder Schadpilzen zu schützenden Saatgüter, Pflanzen, Materialien oder den Erdboden mit einer wirksamen Menge der Wirkstoffe behandelt.

Die Anwendung erfolgt vor oder nach der Infektion der Materialien, Pflanzen oder Samen durch die Schadpilze.

Die Aufwandmengen liegen je nach Art des gewünschten Effektes zwischen 0,02 und 3 kg Wirkstoff pro ha, vorzugsweise bei 0,1 bis 1 kg/ha.

Bei der Saatgutbehandlung werden im allgemeinen Wirkstoffmengen von 0,001 bis 50 g, vorzugsweise 0,01 bis 10 g je Kilogramm Saatgut benötigt.

Die Aufwandmenge an Wirkstoff für die Bekämpfung von Schädlingen oder Schadpilzen beträgt unter Freilandbedingungen 0,02 bis 10, vorzugsweise 0,1 bis 2,0 kg/ha Wirkstoff.

Die Verbindungen I, allein oder in Kombination mit Herbiziden oder Fungiziden, können auch noch mit weiteren Pflanzenschutzmitteln gemischt gemeinsam ausgebracht werden, beispielsweise mit Wachstumsregulatoren oder mit Mitteln zur Bekämpfung von Schädlingen oder Bakterien. Von Interesse ist ferner die Mischbarkeit mit Düngemitteln oder mit Mineralsalzlösungen, welche zur Behebung von Ernährungs- und Spurenelementmängeln eingesetzt werden.

Die Pflanzenschutz- und Düngemittel können zu den erfindungsgemäßen Mitteln im Gewichtsverhältnis 1:10 bis 10:1 zugesetzt werden, gegebenenfalls auch erst unmittelbar vor der Anwendung (Tankmix). Beim Vermischen mit Fungiziden oder Insektiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylen-bis-dithiocarbamat), N,N'-Polypropylen-bis-(thiocarbamoyl)-disulfid; Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-β-[bis-(dimethylamino)-phosphinyl]-3-phenyl-1,2,4-triazol, 2,3-Dicyano-1,4-dithioanthrachinon, 2-Thio-1,3-dithiolo-β-[4,5-b]chinoxalin, 1-(Butylcarbamoyl)-2-benzimidazol-carbaminsäuremethylester, 2-Methoxycarbonylaminobenzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid, N-Dichlorfluormethylthio-N',N'-dimethyl-N-phenylschwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäureanilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclodedecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alaninmethylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin-2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethylphenyl)-5-trifluormethyl-3-chlor-2-aminopyridin, 1-((bis-(4-Fluorphenyl)-methylsilyl)-methyl)-1H-1,2,4-triazol.

### Synthesebeispiele

Die in den nachstehenden Synthesebeispielen wiedergegebenen Vorschriften wurden unter entsprechender Abwandlung der Ausgangsverbindungen bzw. Zwischenprodukte zur Gewinnung weiterer Verbindungen I benutzt. Die so erhaltenen Verbindungen sind in den anschließenden Tabellen mit physikalischen Daten aufgeführt.

### Beispiel 1

### 2-Isopropoxy-6-trifluormethyl-4-hydroxypyrimidin

Zu 357,8 g O-Isopropylisoharnstoff-Hydrochlorid (oder O-Isopropyluroniumchlorid) in 906 ml absolutem Ethanol werden bei Raumtemperatur 465 g 30 %ige methanolische Natriummethanolat-Lösung zugetropft. Nach weiteren 10 min tropft man hierzu unter leicht exothermer Reaktion 475,2 g Trifluoracetessigsäureethylester. Nach 12 h Erwärmen unter Rückfluß engt man den Ansatz im Rotationsverdampfer ein und nimmt den Rückstand in 1 Liter Wasser auf. Die Wasserphase wir mit Salzsäure schwach sauer gestellt und dann mit Methyl-tert.butylether extrahiert. Die vereinigten Etherphasen werden mit Wasser gewaschen, über Natriumsulfat getrocknet und abschließend zur Trockene eingeengt. Der Rückstand wird mit Petrolether verrührt, abgesaugt und getrocknet.

Man erhält 294,5 g der Titelverbindung als farblose Kristalle.
Fp. 126-127°C
¹H-NMR (CDCl₃, δ in ppm):
1,4 (6H); 5,9 (1H); 6,5 (1H); 12,2 (1H)

### Beispiel 2

### 3-Methoxy-2-[2-methylsulfonyl-6-trifluormethyl-pyrimidin-4-yloxymethyl-phenyl]-acrylsäuremethylester (Tab. I.04)

Zu einer Suspension von 3,9 g 3-Methoxy-2-[2-methylthio-6-trifluormethyl-pyrimidin-4-yloxymethyl)-phenyl]-acrylsäuremethylester in 19 ml Eisessig fügt man 0,15 g Dinatriumwolframat·2H₂O hinzu und tropft bei 20-30°C langsam 2,8 ml 30 %ige wäßrige H₂O₂ Lösung zu. Der Ansatz klart innerhalb 1 h auf und wird noch 12 h bei Raumtemperatur gerührt. Zur Aufarbeitung gießt man auf 100 ml Eiswasser, dekantiert nach ca. 30 min den Überstand ab und nimmt den verbleibenden zähen Feststoff in Essigsäureethylester auf. Diese organische Phase wird mit Wasser gewaschen, über Natriumsulfat getrocknet und abschließend eingeengt. Es verbleiben 4,0 g der Titelverbindung als hellgelbes Öl.
¹H-NMR (CDCl₃, δ in ppm):
3,3 (3H); 3,65 (3H); 3,85 (3H); 5,5 (2H); 7,15 (1H); 7,2 (1H); 7,4 (2H); 7,55 (1H); 7,6 (1H)

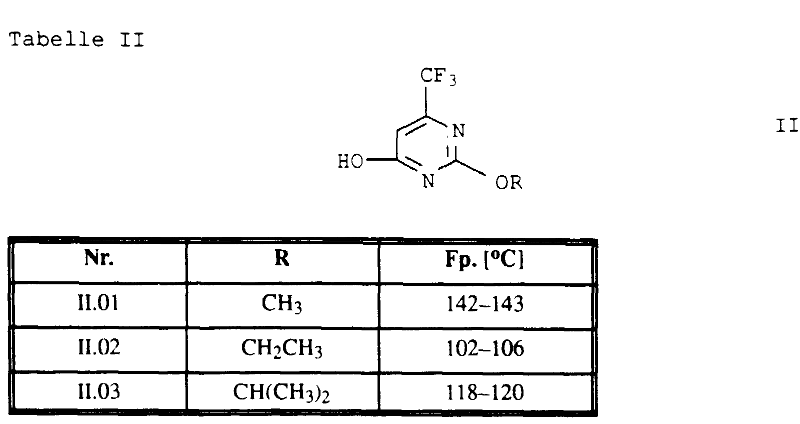

### Beispiel 3

### 3-Methoxy-2-[2-(2-isopropoxy-6-trifluormethyl-pyrimidin-4-yloxymethyl)-phenyl]-acrylsäuremethylester (I.03)

Zur Herstellung des Kaliumsalzes des Hydroxypyrimidins aus Beispiel 1 werden 222 g Hydroxyverbindung in 855 ml Ethanol gelöst und zu einer Lösung von 56 g Kaliumhydroxid in 855 ml Ethanol getropft. Nach 3stündigem Erhitzen unter Rückfluß wird der Ansatz eingeengt und der Rückstand in 2 1 N,N-Dimethylformamid aufgenommen. Zu dieser Lösung des Kaliumsalzes gibt man 280 g 3-Methoxy-2-[2-brommethylphenyl]-acrylsäuremethylester und rührt anschließend 12 h bei 60°C. Zur Aufarbeitung gießt man auf Eiswasser und extrahiert nochmals mit Methyl-tert.-butylether. Die vereinigten Etherphasen werden mit Wasser gewaschen, über Natriumsulfat getrocknet und abschließend eingeengt. Zur Abtrennung eines N-alkylierten Nebenproduktes wird der Rückstand in 1 Liter Methanol gelöst und mit 250 ml Wasser versetzt. Der kristalline Niederschlag wird abgesaugt, mit Petrolether gewaschen und getrocknet. Man erhält 211 g der Titelverbindung.
Fp. 107°C
¹H-NMR (CDCl₃, δ in ppm) :
1,4 (6H); 3,7 (3H); 3,8 (3H); 5,3 (1H); 5,35 (2H); 6,65 (1H); 7,2 (1H): 7,4 (2H); 7,5 (1H); 7,55 (1H)

### Beispiel 4

### 2-Methoxyimino-2-[3 chlor-2-(2-isopropoxy-6-trifluormethylpyrimidin-4-yloxy-methyl)-phenyl]-essigsäuremethylester (I.08)

3,3 g 2-Isopropyloxy-4-hydroxy-6-trifluormethylpyrimidin und 2,4 g Kaliumcarbonat werden in 100 ml Dimethylformamid 30 min. bei Raumtemperatur (ca. 25°C) gerührt und anschließend tropfenweise innerhalb 1 Std. mit einer Lösung aus 4,8 g 2-Methoxyimino-2-[2-brommethyl-3-chlorphenyl]-essigsäuremethylester in 30 ml Dimethylformamid versetzt. Nach weiteren 8 Std. bei Raumtemperatur gibt man die Reaktionsmischung in Eiswasser und extrahiert das Produkt mit tert.-Butyl-methylether. Die vereinigten Etherphasen werden getrocknet und eingeengt. Das so erhaltene Rohprodukt wird chromatographisch (Kieselgel/Toluol) gereinigt. Man erhält 4,2 g der Titelverbindung.
Fp. 106-108°C
1H-NMR (CDCl₃, δ in ppm):
1,4 (6H); 3,85 (3H); 4,0 (3H); 5,35 (1H); 5,45 (1H); 6,6 (1H); 7,1 (1H); 7,4 (1H); 7,55 (1H)

### Beispiel 5

### 2-Methoxyimino-2-[3-chlor-2-(2-isopropyloxy-6-trifluormethyl-pyrimidin-4-yloxymethyl)-phenyl]-essigsäuremethylamid (I.11)

Eine Mischung aus 2,0 g des Methylesters aus Beispiel 4 und 70 ml Tetrahydrofuran werden mit 2 ml einer 40%-igen wäßrigen Methylamid-Lösung versetzt. Zu der Reaktionsmischung gibt man nach 8 Std. bei Raumtemperatur (ca. 25°C) 100 ml tert.-Butyl-methylether. Die so erhaltene Mischung wird 3mal mit 20%-iger Citronensäure und 2 mal mit Wasser gewaschen. Die organische Phase wird getrocknet und eingeengt. Das so erhaltene Rohprodukt wird chromatographisch [Kieselgel/Toluol:Essigsäureethylester (9:1)] gereinigt. Man erhält 1,0g der Titelverbindung.
Fp. 116-118°C
1H-NMR: (CDCl₃, δ in ppm):
1,4 (6H); 2,95 (3H); 3,9 (3H); 5,35 (1H): 5,45 (1H); 6,6 (1H); 6,8 (NH); 7,1 (1H); 7,35 (1H); 7,5 (1H)

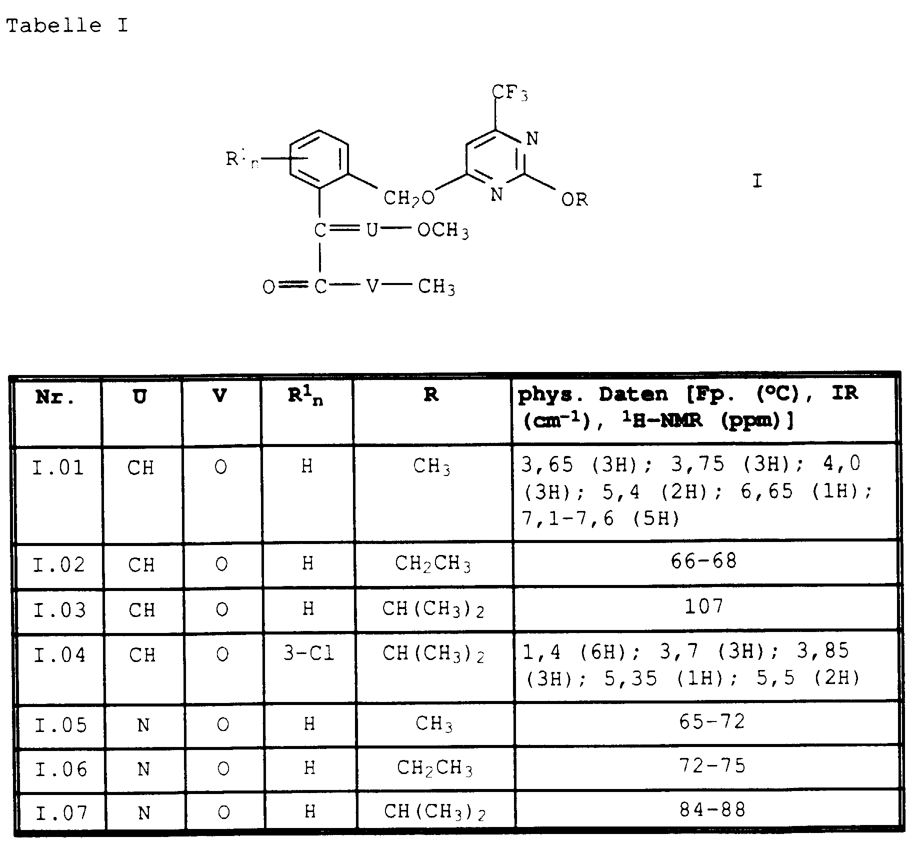

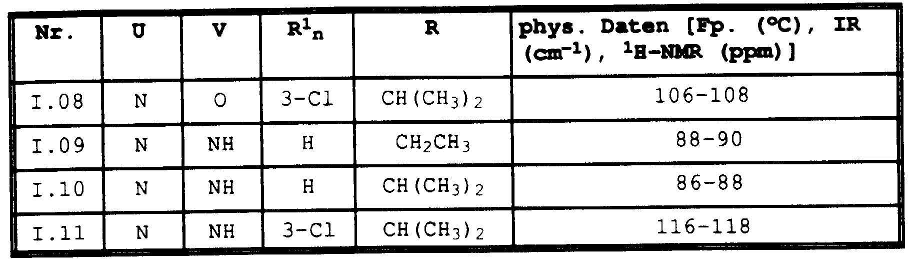

### Beispiele zur Wirkung gegen tierische Schädlinge

Die Wirkung der Verbindungen der allgemeinen Formel I gegen tierische Schädlinge ließ sich durch folgende Versuche zeigen: Die Wirkstoffe wurden
a) als 0,1 %-ige Lösung in Aceton oder
b) als 10 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).
Tetranychus telarius (Rote Spinne), Kontaktwirkung

Stark befallene getopfte Buschbohnen, die das zweite Folgeblattpaar zeigten, wurden mit wässrigen Wirkstoffaufbereitung behandelt. Nach 5 Tagen im Gewächshaus wurde der Bekämpfungserfolg mittels Binokular bestimmt.

In diesem Test zeigten die Verbindungen I.01-I.07 und I.09-I.11 Wirkschwellen von 2 bis 400 ppm.

Nephotettix cincticeps (Grüne Reiszikade), Kontaktwirkung Rundfilter wurden mit der wäßrigen Wirkstoffaufbereitung behandelt und anschließend mit 5 adulten Zikaden belegt. Nach 24 h wurde die Mortalität beurteilt.

In diesem Test zeigten die Verbindungen I.07 und I.08 Wirkschwellen von 0,4 mg.

### Beispiele für die Wirkung gegen Schadpilze

Die fungizide Wirkung der Verbindungen der allgemeinen Formel I ließ sich durch die folgenden Versuche zeigen:

Die Wirkstoffe wurden als 20%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

Als Vergleichsverbindung diente der Wirkstoff **A** (Beispiel Nr. 25, Tabelle I der EP-A 407 872).

### Wirkung gegen Plasmopara viticola (Rebenperonospora)

Topfreben (Sorte: "Müller Thurgau") wurden mit der Wirkstoffaufbereitung tropfnaß gespritzt. Nach 8 Tagen wurden die Pflanzen mit einer Zoosporenaufschwemmung des Pilzes *Plasmopara viticola* besprüht und 5 Tage bei 20-30°C bei hoher Luftfeuchtigkeit bewahrt. Vor der Beurteilung wurden die Pflanzen danach für 16 h bei hoher Luftfeuchtigkeit bewahrt. Die Auswertung erfolgte visuell.

In diesem Test zeigten die mit 250 ppm der erfindungsgemäßen Verbindungen I.01-I.07, I.10 und I.11 behandelten Pflanzen einen Befall von 5% und weniger während die mit der gleichen Menge des bekannten Wirkstoffs **A** behandelten Pflanzen zu 25% befallen waren. Die unbehandelten (Kontroll-) Pflanzen waren zu 75% befallen.

### Wirkung gegen Pyricularia oryzae (Reisbrand)

Reiskeimlinge (Sorte: "Tai Nong 67") wurden mit der Wirkstoffaufbereitung tropfnaß gespritzt. Nach 24 Stunden wurden die Pflanzen mit einer wäßrigen Sporensuspension des Pilzes *Pyricularia oryzae* besprüht und 6 Tage bei 22-24°C bei einer relativen Luftfeuchtigkeit von 95-99 % bewahrt. Die Beurteilung erfolgte visuell.

In diesem Test zeigten die mit 250 ppm der erfindungsgemäßen Verbindungen I.01, I.02, I.04, I.06-I.08, I.10 und I.11 behandelten Pflanzen einen Befall von 25% und weniger während die mit der gleichen Menge des bekannten Wirkstoffs **A** behandelten Pflanzen zu 60% befallen waren. Die unbehandelten (Kontroll-) Pflanzen waren zu 85% befallen.

## Patentansprüche

1. 2-[(2-Alkoxy-6-trifluormethylpyrimidin-4-yl)-oxymethylen]-phenylessigsäurederivate der Formel I 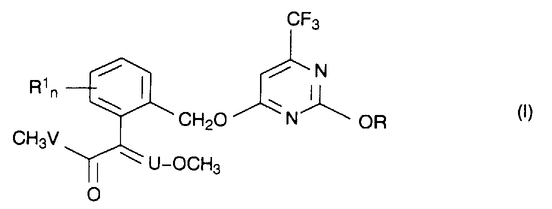 in der der Index und die Substituenten die folgende Bedeutung haben:
U CH oder N;
V O oder NH;
R C₁-C₆-Alkyl;
R¹ Cyano, Halogen, C₁-C₄-Alkyl, C₁-C₄-Halogenalkyl, C₁-C₄-Alkoxy, C₁-C₄-Halogenalkoxy und Phenyl;
n 0 oder eine ganze Zahl von 1 bis 4, wobei die Reste R¹ verschieden sein können, wenn der Wert von n größer als 1 ist.

2. Verbindungen der Formel I gemäß Anspruch 1, in denen U für CH und V für O steht.

3. Verbindungen der Formel I gemäß Anspruch 1, in denen U für N und V für O steht.

4. Verbindungen der Formel I gemäß Anspruch 1, in denen U für N und V für NH steht.

5. Verfahren zur Herstellung der verbindungen I gemäß Anspruch 1, dadurch gekennzeichnet, daß man ein Pyrimidin-4-ol der Formel II 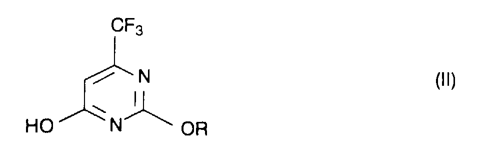 in an sich bekannter Weise in einem inerten organischen Lösungsmittel in Gegenwart einer Base mit einem Benzylderivat der Formel III 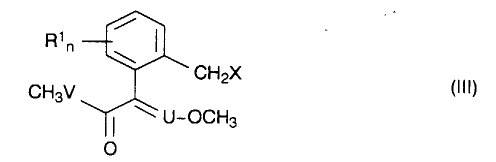 in der X für eine nucleophil austauschbare Abgangsgruppe steht, umsetzt.

6. Verfahren zur Herstellung der Verbindungen I gemäß Anspruch 1, dadurch gekennzeichnet, daß man ein Sulfonderivat der Formel IV 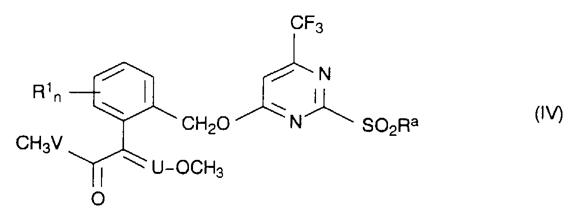 in der R^{a} für C₁-C₄-Alkyl steht, in Gegenwart einer Base mit einem Alkohol der Formel V
HO-R (V)
umsetzt.

7. Verwendung der Pyrimidin-4-ole der Formel II gemäß Anspruch 5 als Zwischenprodukte zur Herstellung von Verbindungen der Formel I gemäß Anspruch 1.

8. Sulfonderivate der Formel IV gemäß Anspruch 6.

9. Verwendung der Sulfonderivate der Formel IV gemäß Anspruch 6 als Zwischenprodukte.

10. Zur Bekämpfung von tierischen Schädlingen und Schadpilzen geeignetes Mittel, enthaltend einen festen oder flüssigen Trägerstoff und eine Verbindung der Formel I gemäß Anspruch 1.

11. Verfahren zur Bekämpfung von tierischen Schädlingen und Schadpilzen, dadurch gekennzeichnet, daß man die Schädlinge bzw. Schadpilze oder die von ihnen zu schützenden Materialien, Pflanzen, Boden oder Saatgüter, ausgenommen am menschlichen oder tierischen körper, mit einer wirksamen Menge einer Verbidnung der Formel I gemäß Anspruch 1 behandelt.

12. Verwendung der Verbindungen der Formel I gemäß Anspruch 1 zur Herstellung eines zur Bekämpfung von tierischen Schädlingen oder Schadpilzen geeigneten Mittels.

13. Verwendung der Verbindungen der Formel I gemäß Anspruch 1 zur Bekämpfung von tierischen Schädlingen oder Schadpilzen, ausgenommen am menschlichen oder tierischen körper.

## Claims

1. A 2-[(2-alkoxy-6-trifluoromethylpyrimidin-4-yl)oxymethylene]-phenylacetic acid derivative of the formula I 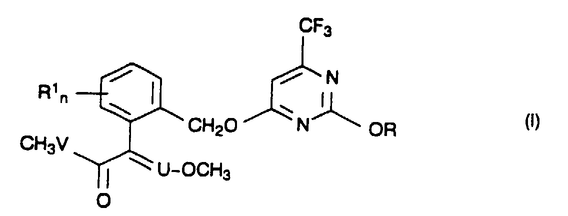 where the index and the substituents have the following meanings:
U is CH or N;
V is O or NH;
R is C₁-C₆-alkyl;
R¹ is cyano, halogen, C₁-C₄-alkyl, C₁-C₄-haloalkyl, C₁-C₄-alkoxy, C₁-C₄-haloalkoxy or phenyl;
n is 0 or an integer from 1 to 4, it being possible for the radicals R¹ to be different if the value of n is greater than 1.

2. A compound of the formula I as claimed in claim 1, where U is CH and V is O.

3. A compound of the formula I as claimed in claim 1, where U is N and V is O.

4. A compound of the formula I as claimed in claim 1, where U is N and V is NH.

5. A process for preparing the compounds I as claimed in claim 1, which comprises reacting a pyrimidin-4-ol of the formula II 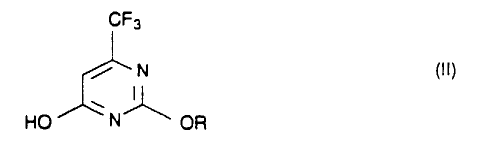 in a manner known per se in an inert organic solvent in the presence of a base with a benzyl derivative of the formula III 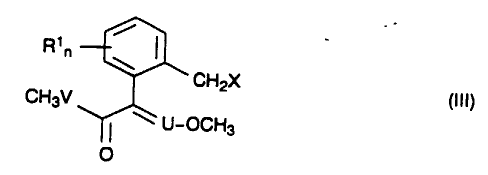 where X is a nucleophilically replaceable leaving group.

6. A process for preparing the compounds I as claimed in claim 1, wherein a sulfone derivative of the formula IV 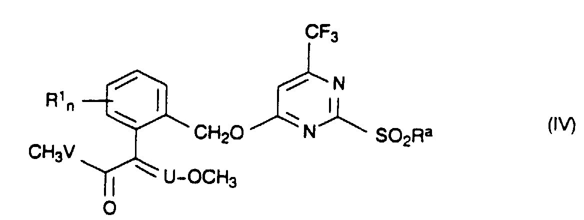 where R^{a} is C₁-C₄-alkyl, is reacted in the presence of a base with an alcohol of the formula V
HO-R (V).

7. The use of the pyrimidin-4-ols of the formula II as set forth in claim 5 as intermediates for the preparation of compounds of the formula I as claimed in claim 1.

8. A sulfone derivative of the formula IV as set forth in claim 6.

9. The use of the sulfone derivatives of the formula IV as set forth in claim 6 as intermediates.

10. A composition suitable for controlling animal pests and harmful fungi, containing a solid or liquid carrier and a compound of the formula I as claimed in claim 1.

11. A process for controlling animal pests and harmful fungi, excepting on the human or animal body, which comprises treating the pests or harmful fungi or the materials, plants, soil or seed to be protected from them with an effective amount of a compound of the formula I as claimed in claim 1.

12. The use of the compounds of the formula I as claimed in claim 1 for preparing a composition suitable for controlling animal pests or harmful fungi.

13. The use of the compounds of the formula I as claimed in claim 1 for controlling animal pests or harmful fungi excepting on the human or animal body.

## Revendications

1. Dérivés d'acide 2-[(2-alcoxy-6-trifluorométhyl-pyrimidine-4-yl)-oxyméthylène]-phénylacétique de formule I 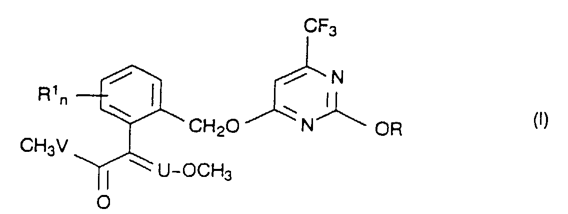 dans laquelle l'indice et les substituants ont la signification ci-après:
U CH ou N;
V 0 ou NH;
R alkyle en C₁-C₆;
R¹ cyano, halogène, alkyle en C₁-C₄, halogénoalkyle en C₁-C₄, alcoxy en C₁-C₄, halogénoalcoxy en C₁-C₄ et phényle;
n 0 ou un nombre entier de 1 à 4, tandis que les restes R¹ peuvent être différents lorsque la valeur de n est supérieure à 1.

2. Composés de formule I selon la revendication 1, dans lesquels U désigne CH et V représente O.

3. Composés de formule I selon la revendication 1, dans lesquels U désigne N et V représente O.

4. Composés de formule I selon la revendication 1, dans lesquels U désigne N et V représente NH.

5. Procédé pour la préparation des composés I selon la revendication 1, caractérisé par le fait qu'on fait réagir un pyrimidine-4-ol de formule II 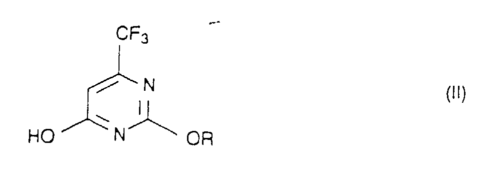 de manière connue en soi dans un solvant inerte en présence d'une base avec un dérivé benzylique de formule III 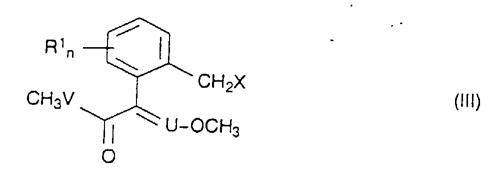 où X désigne un groupe éliminable échangeable par voie nucléophile.

6. Procédé pour la préparation des composés I selon la revendication 1, caractérisé par le fait qu'on fait réagir un dérivé sulfonique de formule IV 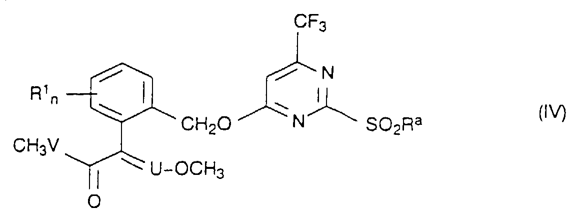 où R^{a} représente un alkyle en C₁-C₄, en présence d'une base avec un alcool de formule V
HO-R (V)

7. Utilisation des pyrimidine-4-ols de formule II selon la revendication 5 comme produits intermédiaires pour la préparation de composés de formule I selon la revendication 1.

8. Dérivés sulfoniques de formule IV selon la revendication 6.

9. Utilisation des dérivés sulfoniques de formule IV selon la revendication 6 comme produits intermédiaires.

10. Agent approprié pour la lutte contre les parasites animaux et les champignons nuisibles, contenant un support solide ou liquide et un composé de formule I selon la revendication 1.

11. Procédé pour la lutte contre les parasites animaux et les champignons nuisibles, caractérisé par le fait qu'on traite les parasites ou les champignons nuisibles ou les matières, plantes, sols ou semences à protéger contre eux, excepté sur le corps humain ou animal, avec une quantité efficace d'un composé de formule I selon la revendication 1.

12. Utilisation des composés de formule I selon la revendication 1 pour la préparation d'un moyen approprié pour la lutte contre les parasites animaux ou les champignons nuisibles.

13. Utilisation des composés de formule I selon la revendication 1 pour la lutte contre les parasites animaux ou les champignons nuisibles, excepté sur le corps humain ou animal.
